# EUROPEAN PATENT SPECIFICATION

(11) **EP 3 429 607 B1**
(45) Date of publication and mention of the grant of the patent: **15.12.2021**
(21) Application number: 17714160.3
(22) Date of filing: 15.03.2017
(51) Int. Cl.: A61K 9/06, A61K 36/77, A61K 36/87, A61K 36/9068, A61K 9/00, A61K 47/36, A61K 36/185, A61K 36/45, A61P 1/02, A61P 1/04, A61P 11/02, A61P 11/04, A61P 31/04, A61P 31/12, A61P 39/00, A61P 31/16, A61P 29/00, A61K 9/20, A61K 9/19

(54) **COMPOSITIONS USEFUL IN THE PREVENTION AND/OR TREATMENT OF DISORDERS OF THE ORAL CAVITY, UPPER AIRWAYS AND ESOPHAGUS**
ZUSAMMENSETZUNGEN ZUR PRÄVENTION UND/ODER BEHANDLUNG VON KRANKHEITEN DER MUNDHÖHLE, OBEREN ATEMWEGE UND SPEISERÖHRE
COMPOSITIONS UTILISABLES DANS LA PRÉVENTION ET/OU LE TRAITEMENT DE TROUBLES DE LA CAVITÉ BUCCALE, DES VOIES RESPIRATOIRES SUPÉRIEURES ET DE L' OESOPHAGE

(30) Priority: 18.03.2016 IT UA20161822
(43) Date of publication of application: 23.01.2019
(73) Proprietor: Indena S.p.A., 20139 Milano (IT)
(72) Inventor: BOMBARDELLI, Ezio, 27027 Gropello Cairoli (PV) (IT)
(74) Representative: Bianchetti & Minoja SRL
(86) International application number: PCT/EP2017/056148
(87) International publication number: WO 2017/158041

(56) References cited:
- EP-A1- 0 914 832
- EP-A1- 2 324 821
- EP-A1- 2 476 421
- WO-A1-00/46252
- WO-A1-2006/063716
- WO-A1-2010/146601
- WO-A1-2012/049045
- WO-A1-2016/004952
- WO-A2-02/09637
- WO-A2-2011/135314
- WO-A2-2013/188819
- WO-A2-2015/017316
- FR-A1- 2 972 327
- US-A1- 2005 226 907
- US-A1- 2009 264 392
- US-A1- 2014 051 731
- US-A1- 2016 051 683
- DATABASE WPI Week 201235 Thomson Scientific, London, GB; AN 2011-K13552 XP002764152, & BE 1 018 740 A3 (OYSTERSHELL NV) 5 July 2011 (2011-07-05)
- DATABASE WPI Week 201618 Thomson Scientific, London, GB; AN 2015-610464 XP002764153, & CN 104 840 958 A (GUANGZHOU DIQI MEDICAL TECHNOLOGY CO LTD) 19 August 2015 (2015-08-19)
- BARNABY S N ET AL: "Formation of hyaluronic acid-ellagic acid microfiber hybrid hydrogels and their applications", COLLOID AND POLYMER SCIENCE 2013 SPRINGER VERLAG DEU, vol. 291, no. 3, March 2013 (2013-03), pages 515-525, XP002770783, ISSN: 0303-402X
- DATABASE WPI Week 201058 Thomson Scientific, London, GB; AN 2010-K38015 XP002770784, & WO 2010/092941 A1 (BOURBON CORP) 19 August 2010 (2010-08-19)
- DATABASE WPI Week 201059 Thomson Scientific, London, GB; AN 2010-K60693 XP002770785, & CN 101 787 214 A (UNIV SICHUAN) 28 July 2010 (2010-07-28)

## Description

### Technical field of the invention

The present invention relates to compositions comprising at least one film-forming agent able to adhere in a stable way to the mucosa of the mouth, esophagus and upper airways, and optionally at least one compound or extract obtained from a medicinal plant having antimicrobial or antiviral activity.

Said compositions are useful in the prevention and/or treatment of disorders of the oral cavity and upper airways caused by bacterial and/or viral agents, and of the esophagus.

### Background of the invention

Reddening, inflammation and bacterial and/or fungal infections of the throat, with plaque formation, are symptoms that commonly accompany influenza, colds and similar disorders.

The common cold and influenza, which affect both children and adults up to three times a year on average, are mainly associated with viral infections, 40% of which are caused by rhinovirus, 10% by coronavirus and a smaller proportion by adenovirus and parainfluenza virus. Although there is no specific treatment for these disorders, antihistamines, decongestants and anti-inflammatories are considered useful, because reduction of oedema alleviates pain and shortens the length of the disorder underlying the inflammation.

These disorders sometimes involve complications due to the onset of secondary bacterial infections, because the outlets of the nasal sinuses are often obstructed due to congestion of the mucosa, where pathogenic germs can easily proliferate, causing fever and localized pain. In this case, antibiotic treatment is required in addition to symptomatic treatments.

One of the aspects to be considered during epidemics is the ease of transmission of the disease through involuntary contact with carriers.

There is still a need to identify alternative products which are useful in the prevention and/or treatment of disorders of the oral cavity, esophagus and upper airways caused by bacterial and/or viral agents and/or chemical agents.

### Summary of the invention

The invention relates to compositions comprising at least one film-forming agent able to adhere in a stable way to the mucosa of the mouth, esophagus and upper airways, obtainable by cross-linking salts of hyaluronic acid or of alginic acid in the presence of anthocyanidins or proanthocyanidins in free or glycosylated form, or in the presence of extracts obtained from medicinal plants containing anthocyanidins or proanthocyanidins.

The invention also relates to compositions for use in the prevention and/or treatment of disorders of the oral cavity, esophagus and upper airways caused by chemical agents and/or bacterial and/or viral agents.

### Detailed description of the invention

The present invention relates to compositions comprising at least one film-forming agent able to adhere in a stable way to the mucosa of the mouth, esophagus and upper airways, obtainable by cross-linking salts of hyaluronic acid or of alginic acid in the presence of anthocyanidins or proanthocyanidins in free or glycosylated form, or in the presence of extracts obtained from medicinal plants containing anthocyanidins or proanthocyanidins.

The composition can optionally contain at least one compound or extract obtained from a medicinal plant having antimicrobial or antiviral activity.

It has now surprisingly been found that compositions comprising at least one film-forming agent able to adhere in a stable way to the mucosa of the mouth, esophagus and upper airways as defined above protect against attack by a chemical agent and/or a bacterial and/or viral agent on the mucosa, thus allowing the prevention and/or treatment of disorders of the oral cavity, esophagus and upper airways caused by chemical agents and/or bacterial and/or viral agents.

The film-forming agent is made from known polymer molecules, i.e. hyaluronic acid or alginic acid,, by reacting them with compounds having a strong protein bond or with extracts obtained from medicinal plants containing said compounds, i.e. anthocyanidins or proanthocyanidins, in free or glycosylated form,.

Extracts suitable for said purpose are extracts of *Vaccinium myrtillus, Vaccinium uliginosum, Punica granatum, Vitis vinifera* and *Aesculus ippocastanum.* The extract is preferably *Vaccinium myrtillus* or *Vaccinium uliginosum* extract. More preferably it is *Vaccinium uliginosum* extract.

According to a preferred aspect, an extract of *Vaccinium myrtillus, Vaccinium uliginosum, Punica granatum, Vitis vinifera* or *Aesculus ippocastanum* may be used in quantities ranging from 40 to 400 mg.

Hyaluronic acid and alginic acid are used, due to their property of generating salts with anthocyanosides and alkaloids or forming new polymers by cross-reactions able to incorporate tannic substances with a high affinity for proteins in the crosslinked structures.

With hyaluronic acid and alginates in salt form with sodium or potassium, anthocyanosides, by double exchange, give rise to new salts wherein the oxonium base bonds the carboxyls of the corresponding polymers, leaving the phenol part exposed; *in vivo,* the phenol part is anchored to the muciparous protein part of the mucosa of the mouth and adnexa, such as the palatine tonsils, Waldeyer's lymphatic ring, the proximal part of the esophagus and the upper airways, and partly to the phospholipid portion.

The barrier effect is so considerable due to the adhesion of the film-forming agent to the tissue, and is far greater than that obtainable by administering the compounds separately.

The film-forming agent reacts with the protein part of the secretion of the muciparous cells to form a protective and active barrier against the aggressive external pathogen.

This reduces bacterial and viral adhesion to the tissues, and the harm which can be caused to mucosa damaged and inflamed by pathogens, acidity or food.

The compositions according to the invention therefore prevent the formation of purulent plaques deriving from saprophytic infections of the oral cavity, thus avoiding the use of antibiotics, especially in infants and the elderly.

Moreover, the compositions according to the invention perform a favourable activity by cleaning the oral cavity reducing bacterial adhesion to the mucosa, with a consequent preventive effect.

The reaction between anthocyanosides and polymer can take place in water or ethanol/water mixtures; the resulting paste-like polymer is then freeze-dried. Natural acidic polymers reacted with binders that retain the polyphenols in the matrices can be advantageously used as an alternative to salts, which are not obtainable with non-cationic molecules.

As reported in the literature, hyaluronic acid and alginic acid, like some pectins, can react with crosslinkers such as divinyl sulfone (DVS), 1,4-butanediol diglycidyl ether, water-soluble carbodiimides (e.g. 1-ethyl-3-(3-dimethylaminopropyl)carbodiimide), crosslinkers with amino groups or urea to give rise to crosslinked polymers which can incorporate other substances in the matrix, in this case polyphenols, which can increase adhesion to the mucosa or other tissues.

Many of said biopolymers are normally compatible with human tissues, but various problems arise. In fact, it has been found that not all these crosslinkers possess adequate biocompatibility with body tissues, especially the mucosa, giving rise to adverse reactions. Biopolymers were therefore selected which, when combined with polyphenols, possess high biocompatibility with the mucosa and skin tissues.

The biopolymer obtainable with urea is preferred. Said crosslinked biopolymer is a sufficiently fluid, physiologically acceptable macromolecular matrix. The end product is obtainable by reacting hyaluronic acid with urea by acid catalysis, or by other methods reported in the examples.

In some cases, hyaluronic acid salified with sodium or potassium is crosslinked by reacting it with urea, and a compound characterised by possessing two amino groups bonded to a carbonyl functional group is obtained.

The hyaluronic acid or alginic acid preferably has a molecular weight ranging between 100,000 and 10,000,000 daltons, more preferably 1,000,000 daltons.

Hyaluronic or alginic acid can normally dissolve in water in amounts ranging from 0.5 to 5% w/w, and urea in amounts ranging from 0.2 to 2% w/w, using dilute mineral acids such as sulphuric or hydrochloric acid, mainly dilute sulphuric acid, for the acid catalysis.

In the case of crosslinking, the polymers are characterised by analysis of viscosity η and shear stress τ as a function of shear rate γ by methods extensively reported in the literature, or by IR determination. The procedure anyhow establishes the consistency and applicability of the polymer to the tissues to be treated.

According to a preferred aspect, the compositions according to the present invention may further comprise at least one extract obtained from a medicinal plant having antimicrobial or antiviral activity, for example able to inhibit replication of the pathogens that usually cause colds and influenza, such as an essential oil. The extract obtained from a medicinal plant having antimicrobial or antiviral activity is preferably a lipophilic extract of *Zingiber officinale.*

The lipophilic extract of *Zingiber officinale* is preferably obtained from the roots and rhizomes.

The compositions may comprise the lipophilic extract of *Zingiber officinale* in amounts ranging from 0.01% w/w to 1% w/w, preferably in amounts ranging from 0.1% w/w to 0.8% w/w, and even more preferably in amounts of 0.5% w/w.

The lipophilic extract of *Zingiber officinale* can be obtained by extraction from the roots or rhizomes with alcohols, ketones or aliphatic ethers or, preferably, with carbon dioxide under supercritical conditions as described in EP0464298 A1 (page 2 lines 1-52, and page 5 line 45 to page 6 line 7).

According to a further aspect, the compositions may comprise excipients designed to make their flavour, and consequently their administration, pleasant.

The compositions according to the invention can be prepared by well-known methods, such as those described in "Remington's Pharmaceutical Handbook", Mack Publishing Co., N.Y., USA.

The compositions according to the invention may preferably be formulated in the form of tablets that dissolve slowly in the oral cavity, in suitable chewing gum forms, or in the form of gels to be held in the mouth, in such a way as to guarantee a reasonable time for adhesion to the mucosa so as to form a barrier against both pathogens and mechanical injuries.

A further object of the present invention is the use of compositions comprising at least one film-forming agent able to adhere in a stable way to the mucosa of the mouth, esophagus and upper airways, as defined above and optionally at least one compound or extract obtained from a medicinal plant having antimicrobial or antiviral activity, in the prevention and/or treatment of disorders of the oral cavity, esophagus and upper airways caused by chemical agents and/or bacterial and/or viral agents.

The disorders caused by chemical agents and/or bacterial and/or viral agents may be, for example, colds, influenza, oral and esophageal mucositis of various origins, damages to the esophageal mucosa induced by hyperacidity and esophageal reflux.

It has been found that by administering the compositions according to the invention, considerable, long-lasting adhesion thereof to the mucosa is obtained. A barrier effect is thus achieved that protects the oropharyngeal/esophageal tract against attack by acids and enzymes due to reflux, and reduces the extent of the above-mentioned damage. This result is very useful in view of the high percentage of individuals suffering from said disorder.

Moreover, the barrier effect keeps bacterial and viral proliferation under control, thus reducing the transmission of pathogens at oropharyngeal level.

The examples below further illustrate the invention.

### Example 1 - Preparation of crosslinked hyaluronic acid in the presence of Vaccinium uliginosum extract containing 30% cyanidin-3-O-glucoside C1

16 g of hyaluronic acid potassium salt (1 million daltons) are dissolved in 200 ml of distilled water together with 5 g of *Vaccinium uliginosum* extract having a 30% cyanidin-3-glucoside content and a 35% procyanidin content. A solution of 20 g of urea in 100 ml of 1.4% hydrochloric acid is added slowly to said solution. The mixture is left under stirring for 12 h at 25°C. A bright red rubbery mass forms, which can be separated from the reaction medium by centrifugation to eliminate the excess urea and salts. The gelatinous residue is freeze-dried, and the resulting product in powder form can be used to prepare gels or orodispersible tablets.

### Example 2 - Preparation of crosslinked alginic acid in the presence of procyanidins obtained from Vitis vinifera

12 g of sodium alginate (250,000 D) are dissolved in 250 ml of saline solution; 8 g of *Vitis vinifera* extract containing 90% procyanidins are added, and after homogenization, 20 g of urea dissolved in 200 ml of physiological saline solution are added under stirring. The mixture is treated with 20 ml of 0.5 N hydrochloric acid. The strongly gelled solution is left to stand for 12 hours. The gel is then buffered with NaOH and poured under stirring into 500 ml of ethanol. The solid is centrifuged, then collected and freeze-dried. This product can be used in solid formulations or suitably diluted to form gels.

### Example 3 - Preparation of crosslinked alginic acid in the presence of Vaccinium myrtillus extract and Punica granatum extract.

12 g of sodium alginate (250,000 D) are dissolved in 250 ml of physiological saline solution; 4 g of *Punica granatum* extract containing 45% punicalagin and 3 g of *Vaccinium myrtillus* extract containing 36% anthocyanosides are added, and after homogenisation, 20 g of urea dissolved in 200 ml of physiological saline solution are added under stirring. The mixture is treated with 20 ml of 0.5 N hydrochloric acid. The strongly gelled solution is left to stand for 12 hours. The gel is then buffered with NaOH and poured under stirring into 500 ml of ethanol. The solid is centrifuged, then collected and freeze-dried. This product can be used in solid formulations or suitably diluted to form gels.

### Example 4 - Preparation of orodispersible tablets

| | |
|---|---|
| Crosslinked compound obtained in example 1 | 200 mg |
| Lipophilic extract of *Zingiber officinale* | 5 mg |
| Xylitol | 400 mg |
| Ammonium glycyrrhizinate | 10 mg |
| Sodium cyclamate | 40 mg |
| Polysorbate 80 | 5 mg |

### Example 5 - Preparation of orodispersible tablets

| Crosslinked compound obtained in example 26 | 200 mg |
|---|---|
| Lipophilic extract of *Zingiber officinale* | 5 mg |
| Xylitol | 400 mg |
| Ammonium glycyrrhizinate | 10 mg |
| Sodium cyclamate | 40 mg |
| Polysorbate 80 | 5 mg |

## Claims

1. Compositions comprising at least one film-forming agent able to adhere in a stable manner to the mucosa of oral cavity, esophagus and upper airways, obtainable by cross-linking salts of hyaluronic acid or of alginic acid in the presence of anthocyanidins or proanthocyanidins in free or glycosylated form, or in the presence of extracts obtained from medicinal plants containing anthocyanidins or proanthocyanidins.

2. Compositions according to claim 1, wherein the hyaluronic acid or alginic acid has a molecular weight ranging from 100,000 to 10,000,000 daltons, preferably 1,000,000 daltons.

3. Compositions according to claim 1 or 2, wherein the extracts are selected from extracts of *Vaccinium myrtillus, Vaccinium uliginosum, Punica granatum, Vitis vinifera* and *Aesculus ippocastanum.*

4. Compositions according to claim 3, wherein the extract is a *Vaccinium myrtillus, Vaccinium uliginosum, Punica granatum or Vitis vinifera* extract.

5. Compositions according to claims 1-4, wherein the hyaluronic or alginic acid is crosslinked with crosslinking agents selected from divinyl sulfone (DVS), 1,4-butanediol diglycidyl ether, water-soluble carbodiimides, crosslinking agents with amino groups and urea.

6. Compositions according to claim 5, wherein the cross-linking agent is urea.

7. Compositions according to claims 1-6, further comprising at least one compound or extract obtained from a medicinal plant having an antimicrobial or antiviral activity.

8. Compositions according to claim 7, wherein the extract obtained from a medicinal plant having an antimicrobial or antiviral activity is a lipophilic extract of *Zingiber officinale.*

9. Compositions according to claim 8, wherein the amount of the lipophilic extract of *Zingiber officinale* ranges from 0.01% w/w to 1% w/w, preferably from 0.1% w/w to 0.8% w/w, and is even more preferably 0.5% w/w.

10. Compositions according to claims 1-9 for use in the prevention and/or treatment of disorders of the oral cavity, esophagus and upper airways caused by chemical and/or bacterial and/or viral agents.

11. Compositions for use according to claim 10, wherein the disorders caused by chemical and/or bacterial and/or viral agents are colds, influenza, oral and esophageal mucositis of various origins, and damages to the esophageal mucosa induced by hyperacidity and esophageal reflux.

12. A process for preparing a composition according to claims 1-9, said process comprising cross-linking salts of hyaluronic acid or of alginic acid in the presence of anthocyanidins or proanthocyanidins in free or glycosylated form or in the presence of extracts obtained from medicinal plants containing anthocyanidins or proanthocyanidins.

13. The process according to claim 12, wherein the extract is selected from a *Vaccinium myrtillus, Vaccinium uliginosum, Punica granatum* or *Vitis vinifera* extract.

14. The process according to claim 12 or 13, wherein the cross-linking occurs in the presence of a cross-linking agent selected from divinyl sulfone (DVS), 1,4-butanediol diglycidyl ether, water-soluble carbodiimides, crosslinking agents with amino groups and urea.

15. The process according to claim 14, wherein the cross-linking occurs in the presence of urea.

## Patentansprüche

1. Zusammensetzungen, die mindestens ein filmbildendes Mittel umfassen, das in der Lage ist, in stabiler Weise an der Schleimhaut der Mundhöhle, der Speiseröhre und der oberen Atemwege zu haften, erhältlich durch Vernetzung von Salzen der Hyaluronsäure oder der Alginsäure in Gegenwart von Anthocyanidinen oder Proanthocyanidinen in freier oder glykosylierter Form oder in Gegenwart von Extrakten, erhalten aus Heilpflanzen, die Anthocyanidine oder Proanthocyanidine enthalten.

2. Zusammensetzungen nach Anspruch 1, wobei die Hyaluronsäure oder Alginsäure ein Molekulargewicht im Bereich von 100000 bis 10000000 Dalton, vorzugsweise 1000000 Dalton, aufweist.

3. Zusammensetzungen nach Anspruch 1 oder 2, wobei die Extrakte aus Extrakten von *Vaccinium myrtillus, Vaccinium uliginosum, Punica granatum, Vitis vinifera* und *Aesculus ippocastanum* ausgewählt sind.

4. Zusammensetzungen nach Anspruch 3, wobei der Extrakt ein *Vaccinium myrtillus-, Vaccinium uliginosum-, Punica granatum-* oder *Vitis vinifera-Extrakt* ist.

5. Zusammensetzungen nach den Ansprüchen 1-4, wobei die Hyaluron- oder Alginsäure mit Vernetzungsmitteln, ausgewählt aus Divinylsulfon (DVS), 1,4-Butandioldiglycidylether, wasserlöslichen Carbodiimiden, Vernetzungsmitteln mit Aminogruppen und Harnstoff, vernetzt ist.

6. Zusammensetzungen nach Anspruch 5, wobei das Vernetzungsmittel Harnstoff ist.

7. Zusammensetzungen nach den Ansprüchen 1-6, die weiter mindestens eine Verbindung oder einen Extrakt, erhalten aus einer Heilpflanze mit antimikrobieller oder antiviraler Wirkung, umfassen.

8. Zusammensetzungen nach Anspruch 7, wobei der aus einer Heilpflanze mit antimikrobieller oder antiviraler Wirkung erhaltene Extrakt ein lipophiler Extrakt von *Zingiber officinale* ist.

9. Zusammensetzungen nach Anspruch 8, wobei die Menge des lipophilen Extrakts von *Zingiber officinale* im Bereich von 0,01 % (Gew./Gew.) bis 1 % (Gew./Gew.), vorzugsweise von 0,1 % (Gew./Gew.) bis 0,8 % (Gew./Gew.) und bevorzugter 0,5 % (Gew./Gew.) liegt.

10. Zusammensetzungen nach den Ansprüchen 1-9 zur Verwendung bei der Prävention und/oder Behandlung von Erkrankungen der Mundhöhle, der Speiseröhre und der oberen Atemwege, die durch chemische und/oder bakterielle und/oder virale Mittel verursacht werden.

11. Zusammensetzungen zur Verwendung nach Anspruch 10, wobei die durch chemische und/oder bakterielle und/oder virale Mittel verursachten Erkrankungen Erkältungen, Grippe, Mund- und Speiseröhrenschleimhautentzündungen verschiedenen Ursprungs und durch Übersäuerung und Speiseröhrenreflux hervorgerufene Schäden an der Speiseröhrenschleimhaut sind.

12. Verfahren zur Herstellung einer Zusammensetzung nach den Ansprüchen 1-9, wobei das Verfahren die Vernetzung von Salzen der Hyaluronsäure oder Alginsäure in Gegenwart von Anthocyanidinen oder Proanthocyanidinen in freier oder glykosylierter Form oder in Gegenwart von Extrakten aus Heilpflanzen, die Anthocyanidine oder Proanthocyanidine enthalten, umfasst.

13. Verfahren nach Anspruch 12, wobei der Extrakt aus einem *Vaccinium myrtillus-, Vaccinium uliginosum-, Punica granatum-* oder *Vitis vinifera-Extrakt* ausgewählt ist.

14. Verfahren nach Anspruch 12 oder 13, wobei die Vernetzung in Gegenwart eines Vernetzungsmittels, ausgewählt aus Divinylsulfon (DVS), 1,4-Butandioldiglycidylether, wasserlöslichen Carbodiimiden, Vernetzungsmitteln mit Aminogruppen und Harnstoff, erfolgt.

15. Verfahren nach Anspruch 14, wobei die Vernetzung in Gegenwart von Harnstoff erfolgt.

## Revendications

1. Compositions comprenant au moins un agent formant film capable d'adhérer d'une manière stable à la muqueuse de la cavité buccale, à l'œsophage et aux voies respiratoires supérieures, pouvant être obtenues par la réticulation de sels d'acide hyaluronique ou d'acide alginique en présence d'anthocyanidines ou de proanthocyanidines sous une forme libre ou glycosylée, ou en présence d'extraits obtenus à partir de plantes médicinales contenant des anthocyanidines ou des proanthocyanidines.

2. Compositions selon la revendication 1, l'acide hyaluronique ou l'acide alginique ayant un poids moléculaire s'étendant de 100 000 à 10 000 000 daltons, préférablement 1 000 000 daltons.

3. Compositions selon la revendication 1 ou 2, les extraits étant sélectionnés parmi les extraits de *Vaccinium myrtillus, Vaccinium uliginosum, Punica granatum, Vitis vinifera* et *Aesculus ippocastanum.*

4. Compositions selon la revendication 3, l'extrait étant un extrait de *Vaccinium myrtillus, Vaccinium uliginosum, Punica granatum ou Vitis vinifera.*

5. Compositions selon les revendications 1 à 4, l'acide hyaluronique ou alginique étant réticulé avec des agents de réticulation sélectionnés parmi la divinyl sulfone (DVS), l'éther diglycidylique de 1,4-butanediol, les carbodiimides solubles dans l'eau, les agents de réticulation avec des groupes amino et l'urée.

6. Compositions selon la revendication 5, l'agent de réticulation étant l'urée.

7. Compositions selon les revendications 1 à 6, comprenant en outre au moins un composé ou un extrait obtenu à partir d'une plante médicinale ayant une activité antimicrobienne ou antivirale.

8. Compositions selon la revendication 7, l'extrait obtenu à partir d'une plante médicinale ayant une activité antimicrobienne ou antivirale étant un extrait lipophile de *Zingiber officinale.*

9. Compositions selon la revendication 8, la quantité de l'extrait lipophile de *Zingiber officinale* s'étendant de 0,01 % en pds/pds à 1 % en pds/pds, préférablement de 0,1 % en pds/pds à 0,8 % en pds/pds, et étant même plus préférablement de 0,5 % en pds/pds.

10. Compositions selon les revendications 1 à 9 pour l'utilisation dans la prévention et/ou le traitement des troubles de la cavité buccale, de l'œsophage et des voies respiratoires supérieures causés par des agents chimiques et/ou bactériens et/ou viraux.

11. Compositions pour l'utilisation selon la revendication 10, les troubles causés par des agents chimiques et/ou bactériens et/ou viraux étant les refroidissements, la grippe, la mucosité buccale et œsophagienne d'origines variées, et les lésions à la muqueuse de l'œsophage induites par l'hyperacidité et le reflux oesophagien.

12. Procédé de préparation d'une composition selon les revendications 1 à 9, ledit procédé comprenant la réticulation de sels d'acide hyaluronique ou d'acide alginique en présence d'anthocyanidines ou de proanthocyanidines sous une forme libre ou glycosylée ou en présence d'extraits obtenus à partir de plantes médicinales contenant des anthocyanidines ou des proanthocyanidines.

13. Procédé selon la revendication 12, l'extrait étant sélectionné parmi un extrait de *Vaccinium myrtillus, Vaccinium uliginosum, Punica granatum* ou *Vitis vinifera.*

14. Procédé selon la revendication 12 ou 13, la réticulation se produisant en présence d'un agent de réticulation sélectionné parmi la divinyl sulfone (DVS), l'éther diglycidylique de 1,4-butanediol, les carbodiimides solubles dans l'eau, les agents de réticulation avec des groupes amino et l'urée.

15. Procédé selon la revendication 14, la réticulation se produisant en présence d'urée.
